# EUROPEAN PATENT SPECIFICATION

(11) **EP 4 452 102 B1**
(45) Date of publication and mention of the grant of the patent: **18.11.2026**
(21) Application number: 22843847.9
(22) Date of filing: 20.12.2022
(51) Int. Cl.: A61B 17/54, A61B 17/32

(54) **METHOD AND DEVICE FOR ABRASION OF OUTER LAYER OF EPIDERMIS**
VERFAHREN UND VORRICHTUNG ZUM ABREIBEN DER ÄUSSEREN EPIDERMISSCHICHT
PROCÉDÉ ET DISPOSITIF D'ABRASION D'UNE COUCHE EXTERNE D'ÉPIDERME

(30) Priority: 23.12.2021 FI 20216342
(43) Date of publication of application: 30.10.2024
(73) Proprietor: Mirka Oy, 66850 Jeppo (FI)
(72) Inventor: KASS, Markus, 68910 Bennäs (FI); HEDE, Hans, 66600 Vörå (FI); BJÖRKMAN, Cecilia, 66900 Nykarleby (FI); CZURYSZKIEWICZ, Teresa, 10300 Karis (FI); FONSEN, Ann-Sofie, 20540 Åbo (FI); RISKU, Charlotta, 65450 Solf (FI); HAGMAN, Johanna, 65100 Vasa (FI); NORRGÅRD, Isabella, 65300 Vasa (FI)
(74) Representative: Kolster Oy Ab
(86) International application number: PCT/FI2022/050850
(87) International publication number: WO 2023/118657

(56) References cited:
- EP-B1- 0 578 865
- WO-A2-2020/252440
- US-A1- 2007 123 808
- US-A1- 2013 345 721

## Description

### TECHNICAL FIELD

The present invention relates to abrasive products, and more particularly to skin abrasion devices and non-therapeutic method of abrading skin.

### BACKGROUND

Hyperkeratosis is a skin condition that thickens a person's skin more than usual in certain places. The thickened layer is made of keratin, which is tough, fibrous protein found in fingernails, hair and skin. The body may produce extra keratin because of inflammation, as a protective response to pressure, or because of a genetic condition.

One type of hyperkeratosis is calluses, which are caused by repeated pressure on a spot of the skin. Extra layers of skin grow over the affected area resulting in harder, raised bulges. Calluses are most often found on heels, palms, toes, and knees. Removal of hyperkeratosis part of the skin can also be applied as a prior treatment for psoriasis or actinic keratosis patients.

A natural remedy for removing hyperkeratosis part of skin is by abrasion. A common problem for using abrasion is how to remove thickened skin efficiently but gently without damaging healthy skin in the process.

US 2013/345721 A1 discloses an article for mechanical skin resurfacing that transfers energy from a handheld motorized device to skin. EP 0 578 865 B1 discloses an abrasive tool and disc comprising an abrasive-containing layer, a fabric substrate for hook and loop attachment to a support plate, and a resilient foam layer between the two.

WO 2020/252440 A2 discloses an abrasive skin care appliance with a facial surface having an abrasive matrix attached.

US 2007/0123808 A1 discloses a cosmetic instrument having various numbers of heads.

### BRIEF DESCRIPTION

An object of the present invention is to provide a solution for above-mentioned problem.

The invention is based on the idea of providing a skin abrasion device and method, which are stated in independent claims. Preferred embodiments are disclosed in dependent claims.

The device of the present disclosure is a cordless skin abrader system consisting of a soft cushioned coated abrasive product, which can be attached to a powered hand-held machine. With a foam cushioned structure of the coated abrasive product, the risk of scarring the skin during abrasion is minimal in comparison to using conventional sandpaper. In addition, when comparing against other existing methods for removal of hyperkeratotic skin, such as cryotherapy, curette, and laser therapy, the device of the present disclosure is easier to use, more time efficient, and gentler. Thereby, the device of the present disclosure contributes to the medical device market for removal of hyperkeratotic skin and stratum corneum as a painless, effective, easy-to-use and time saving device.

### BRIEF DESCRIPTION OF THE DRAWINGS

In the following the invention will be described in greater detail by means of preferred embodiments with reference to the attached drawings, in which
Figure 1 shows a schematic view of a skin abrasion device according to an embodiment;
Figure 2 shows a schematic view of a pad according to an embodiment.

### DETAILED DESCRIPTION

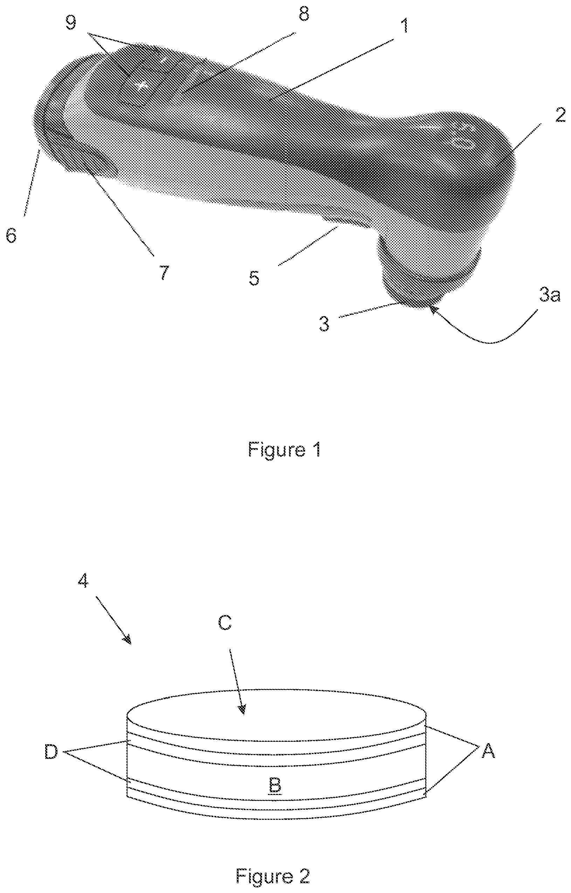

In the following, the invention is described in detail with reference to the drawings. Figure 1 shows a schematic view of a skin abrasion device without an abrasive product. Figure 2 shows a schematic view of the abrasive product.

The present invention pertains to a hand-held, cordless skin abrasion device comprising a handle portion 1 forming part of a housing portion 2, an internal electric drive (not shown in Figures), a backing pad 3, and an attachable abrasive product 4. The device may optionally further comprise a dust extractor suitable for the removal of abrasion skin dust, which may be a vacuum system.

The handle portion 1 may be ergonomically formed, wherein its design features a grip portion. The user may align their fingers in a manner where physical effort and discomfort are minimized. The handle portion 1 may have a cylindrical shape without sharp edges or sharp corners. The surface of the grip portion may be smooth or have an anti-slip configuration.

The embodiment illustrated in Figure 1 comprises a switch 5 for actuating the electric drive. The switch 5 can be a trigger, a lever or a button, for instance. The switch 5 is preferably located underside of the handle portion 1 within easy reach of the user's index finger.

A battery pack 6 may be arranged at an end of the handle portion 1 and preferably partially inside the handle portion 1 and is configured to be removed without the requirement for further tools. The battery pack 6 may be releasably attachable to the handle portion 1 via clamps, for instance. The battery pack 6 may also comprise at least one battery release button 7, preferably two battery release buttons 7 on opposite sides of the battery pack in radial direction. When the user inserts the battery pack 6 inside the handle portion 1, the battery pack 6 may snap-lock in place. When the user presses the battery release button 7, the battery pack 6 is released. The battery pack 6 is preferably a rechargeable battery.

The device may also comprise visual means 8 for determining battery life. The visual means 8 may be for instance one or more LED lights indicating the remaining battery charge. For example, green light indicates good battery charge level and red light indicates low battery charge level. In another example, the visual means consists of several LED lights, in which case all lit LED lights indicate full battery and one lit LED light indicates low battery charge level. The visual means 8 may be located on topside of the handle portion 1, where it is easily visible for the user.

The housing portion 2 may be integrally connected to the handle portion 1 in a gun-like manner, wherein the housing portion 2 has a longitudinal axis and the handle portion 1 has a longitudinal axis which forms an angle of 80-120 degrees with the longitudinal axis of the housing portion 2. The angle is more preferably 90 degrees.

The internal electric drive may locate inside the holding portion 2, and can comprise a motor kit connected to the battery pack 6. The motor kit may comprise an internal motor drive and is arranged to produce oscillating and rotational motion via a counterweight, retaining rings, ball bearings and a balance shaft, for instance. However, other type of systems for producing oscillating and rotational motion can be applied. In this context, the term "oscillating motion" may include a repeatable back and forth movement of the backing pad 3. If present, the back-and-forth movement is preferably in axial direction of the backing pad 3, such as a random orbital or rotating motion of the backing pad 3.

The device is arranged to generate predetermined speed of oscillating motion. The device may further comprise speed selector buttons 9. In the accompanying Figure 1, the speed selector buttons 9 are marked with + and - signs. The user can increase the speed by pressing on the + button and decrease the speed by pressing on the - button.

The backing pad 3 may be attached to the balance shaft with quick-locks and fasteners such as screws, for instance. The backing pad 3 extends from an end of said housing portion 2 and is configured to be set in an oscillating motion by said electric drive. The oscillating motion is 1000 to 15000 rpm (revolutions per minute), preferably 2000 to 12000 rpm, and more preferably 4000 to 8000 rpm by said electric drive. Thanks to the broad spectrum of rpm (1000-15000 rpm) the user is given the opportunity to adjust the speed for a specific kind of surface to be abraded to optimize effectivity and safety.

The device may comprise means for preselecting the rpm of said oscillating motion and/or the length in time of said oscillating motion. In embodiments, the device has an orbital movement, which is gentler than a rotating movement. The gentleness of the orbital movement refers to a uniform speed all over the disc.

In particular, wherein said means comprise means for wireless communication (e.g., using a Bluetooth standard) with a second device and wherein the rpm of said oscillating motion and/or the length in time of said oscillating motion are selected in response to receiving a message defining the rpm of said oscillating motion and/or the length in time of said oscillating motion from said second device via the wireless connection. Said means for wireless communication may be, for instance, a radio transceiver or a receiver. Said second device may be a terminal device or a user equipment, or both. Preselecting a specified maximum running time and/or a constant and/or maximum rpm of said oscillating motion allows for more controlled and higher safety.

The backing pad 3 may comprise an area 3a for the attachable abrasive product 4 to be attached thereon in a mechanical manner. In this context, the term "mechanical manner" refers to a solid, physical type of attachment method which can be manufactured of metal or plastic or composite or mixture thereof. The attachable abrasive product 4 may be attached to said backing pad 3 using a hook and loop system (e.g., Velcro^{®}), in particular wherein the backing pad 3 comprises the hook part of said system, and wherein the abrasive product 4 comprises the loop part of said system positioned at the lower fabric side of said abrasive product 4. In some embodiments, the mechanical manner may include using magnets, vacuum and fasteners, for instance.

In still an embodiment, the abrasive product may comprise a structure as disclosed in WO 2017/174107 A1. In accordance with this embodiment, the abrasive product comprises a knitted cloth on a first side of the flexible abrasive product, grinding agents applied on a second side of the flexible abrasive product facing the first side, and a plurality of loops protruding from the cloth and from the first side, wherein each loop is formed by a pair of bottom-half arcs connected by a protruding head and the loops being arranged in rows extending in the wale direction of the cloth; wherein the bottom-half arcs are interlaced in the cloth, and the protruding heads of the loops are interconnected with one another outside of the cloth so as to form rows of interconnected loops which rows extend in the wale direction of the cloth.

In some embodiments, the abrasive product 4 may be attached to the backing pad 3 in an adhesive manner. In this context, the term "adhesive manner" refers to a non-metallic type of attachment method which comprises a sticky substance. The attachable abrasive product 4 may be attached to said backing pad 3 using an adhesive system, in particular wherein the abrasive product 4 is coated with an adhesive, and the backing pad 3 is adherend. Alternatively, the backing pad 3 may be coated with the adhesive and the abrasive product 4 is the adherend. The adhesive is preferably one of pressure-sensitive adhesives such as elastomer-based compounds. Alternatively, both backing pad 3 and abrasive product 4 may be coated with the adhesive. The adhesive system must allow (i) de- and re-attachment of the abrasive product to the backing pad on the one hand, and (ii) fixation of the abrasive product to the backing pad during the oscillating motion on the other hand.

The backing pad 3 and the abrasive product 4 may each have independently a round shape to which the backing pad 3 and the abrasive product 4 are in contact with each other. The shape is preferably planar and perpendicular to the longitudinal axis of the housing portion 2. The round shape may be in particular a circular shape or an oval shape, and preferably a circular shape. Most preferably the backing pad 3 and the abrasive product 4 may have the same shape. In embodiments, the abrasive product 4 is in the form of an abrasive pad.

The backing pad 3 may have a diameter in the range of 10 to 100 mm, preferably 20 to 77 mm, more preferably 32 to 40 mm, more preferably 32 to 39 mm, more preferably 32 to 38 mm, more preferably 32 to 37 mm, more preferably 32 to 36 mm, more preferably 33 to 35 mm, and most preferably a diameter of about 34 mm at its widest point.

The abrasive product 4 may have a diameter in the range of 10 to 100 mm, preferably 20 to 77 mm, more preferably 32 to 40 mm, preferably 32 to 39 mm, more preferably 32 to 38 mm, more preferably 32 to 37 mm, more preferably 32 to 36 mm, more preferably 33 to 35 mm, and most preferably a diameter of about 34 mm at its widest point.

The diameter of the backing pad 3 may be smaller than the diameter of the abrasive product 4, and wherein the diameter of the backing pad 3 does not differ in more than 4 mm, preferably not more than in 3 mm, more preferably not more than in 2 mm, still more preferably not more than in 1 mm, and most preferably 0,5 mm. Due to the smaller diameter of the backing pad, it is easy to spot if the abrasive product is attached improperly to the backing pad. Generally, the abrasive product should be attached so that the whole backing pad is covered.

The abrasive product 4 may have a thickness of 1 to 10 mm, preferably 4 to 10 mm, more preferably a thickness of 5 to 9 mm, more preferably a thickness of 5 to 8 mm, and even more preferably of 6 to 7 mm.

The abrasive product 4 has a laminated structure with a foam core layer B and a fabric or film layer A (preferably fabric layer A) which is adhered to an upper side of said foam core layer B forming an upper fabric/film side of said abrasive product 4, and to a lower side of said foam core layer B forming a lower fabric side of said abrasive product 4, and said fabric or film layer A (preferably fabric layer A) is each adhered to said foam core layer B by way of an adhesive layer D, and said upper fabric side comprises an abrasive layer C comprising abrasive particles. Thanks to the combination of textile, foam and type of lamination technique, the abrasive particles will bend away from the surface to be abraded when a too high pressure is applied. As a result, the abrasive particles do not cause scarring, but only provide for gentle (but effective) scraping off of the skin surface. As the flexibility of the sanding pad follows the contour of the surface to be abraded, the cushioned foamed sanding pad significantly decrease the risk of scarring the skin in comparison to material containing sharp paper edges as used in conventional abrasive products.

The abrasive product may be a disposable abrasive product. When the abrasive particles have worn out, the abrasive product can be removed and disposed. Likewise, the backing pad may in embodiments be designed to be removed and disposed, when worn out.

The foam core layer B may have a thickness of 1 to 10 mm, preferably 3 to 7 mm, more preferably a thickness of 4 to 6 mm, and most preferably a thickness of about 5 mm.

The foam core layer B may be essentially made of a polymer foam, preferably a synthetic polymer foam, more preferably a polyurethan (PUR) foam, such as a polyether polyurethan (PE-PUR) foam, wherein said foam has a hardness such that it can adapt to the contour of the skin to be abraded. The hotmelt PUR adhesive in layer B may be hardened by drying.

The fabric or film layer A may each independently have a thickness of less than 1 mm, preferably a thickness selected within the range of 0.25 to 0.9 mm, more preferably a thickness selected within the range of 0.4 to 0.8 mm, such as a thickness selected within the range of 0.5 to 0.7 mm.

The fabric layer A may be a woven or non-woven fabric layer made from one or more synthetic polymer fibers, preferably from polyamide fibers.

The abrasive layer C may have a thickness of less than 0.5 mm, in particular a thickness within the range of 0.1 to 0.4 mm, such as a thickness within a range of 0.2 to 0.3 mm.

The abrasive layer C may comprise abrasive particles with a grit number selected within the range of 80 to 4000 according to FEPA, for example 180 to 1000, in particular 360 to 800, such as 500, and/or abrasive particles with an average particle size in diameter of 3 to 201 µm, in particular 20 to 45 µm.

The abrasive particles in said abrasive layer C may be made of silicone carbide (SiC). The abrasive particles may be embedded in said abrasive layer C using a cured resin, e.g. thermally cured resin or radiation cured resin. An example for such a thermally cured resin is phenol formaldehyde resin. Other suitable resins and curing techniques are generally known in the art.

The adhesive layer D may independently each have a thickness of less than 0.5 mm, in particular a thickness within the range of 0.1 to 0.4 mm, such as a thickness within a range of 0.1 to 0.3 mm.

Adhesive techniques may be applied to obtain the adhesive layer D, such as but not limited to flame lamination and dry lamination with thermoplastic adhesive. The adhesive layer D may be a cured hotmelt glue, in particular a cured glue made of a mixture of synthetic plastics, preferably a mixture of synthetic plastics based on polyurethane. The adhesive layer D may alternatively be a thermoplastic adhesive web of a solid material with a nonwoven structure.

A skin abrasion kit may comprise the skin abrasion device and at least one additional abrasive product 4 as replacement. The kit may comprise at least two different abrasive products 4, which differ in their respective grit number and/or average particle size of the abrasive particles, preferably wherein the abrasive particles have grit sizes selected from 180, 360, 500 and 800 according to FEPA.

The kit may further comprise a replacement battery for the device, in particular a rechargeable replacement battery for the device and a charging device for charging the battery of said device.

The kit may further comprise a dust extractor suitable for the removal of abrasion skin dust.

The kit may further comprise user protection equipment, in particular a protective mask and/or gloves.

In addition, the kit may further comprise instructions for use of the device and/or the abrasive product in accordance with the following disclosure concerning methods which apply the device of the present disclosure.

Generally, the device can be used in both non-therapeutic methods and therapeutic methods, dependent on what type of skin is abraded.

Therapeutic methods are not a part of the present invention.

For example, in non-therapeutic methods thickened skin may be removed for cosmetic reasons, e.g. from feet such as to obtain smooth-skinned feet. In this type of use, the device may be used in a similar way than a foot file or corneal file. Likewise, the device may also be used for therapeutic purposes on pathological changes of the skin, as known to the person skilled in the art.

In accordance with the foregoing, the present disclosure provides a method of abrading skin from at least one subject, in particular a non-therapeutic method for abrading skin from at least one subject, comprising the steps of
(a) providing a skin abrasion device as described herein above comprising a first attachable abrasive product with a selected first particle size of the abrasive particles;
(b) conducting a first abrading step, wherein skin is abraded from a subject using the skin abrasion device of step (a);
(c) removing said first attachable abrasive product from said skin abrasion device, and attaching a second attachable abrasive product with a selected second particle size of the abrasive particles; and
(d) conducting a second abrading step, wherein skin is abraded from a subject using the skin abrasion device resulting from step (c).

In preferred embodiments, the skin is human skin. The skin to be abraded is usually cleaned prior to abrasion. Hair removal is not necessary as the hair will gently be removed during abrasion. In each abrading step, the powered device is turned on and the oscillating movements contribute to abrasion of the hyperkeratotic skin as the device is applied parallel onto the skin. The abrasion of skin should be stopped when the hyperkeratotic part of skin has been removed. Examination of completed treatment is mainly conducted by eye-hand examination, but can secondly conducted (e.g. during therapeutic treatments) by examination with a dermatoscope. Remaining skin dust may be removed from the abraded skin with a cleansing wipe.

Method steps (c) and (d) may be repeated at least one more time, such as twice or three times. In a particular embodiment, the subject in step (b) and step (d) is the same subject. In this case the second particle size is usually the same or smaller than the first particle size. Indeed, most often the second particle size is smaller than the first particle size. A suitable abrasive product is often chosen with a grit number between 360 and 1000, according to hardness of the hyperkeratotic skin to be abraded. Typically the harder and the thicker the skin, the lower the grit number. As disclosed herein above, the abrasive particles may have at least grit sizes selected from 80 - 4000 according to FEPA. Grit sizes of 500 and finer are of largest interest for removal of hyperkeratotic skin on sensitive skin areas, whereas grit sizes of 360 and coarser are of largest interest for podiatry. A new abrasive product may also be used when another part of the body is to be abraded. The abrasive product is discarded after use.

In embodiments, the method further comprises after step (a) and prior to step (b), and/or after step (b) and prior to step (d), an additional step of preselecting the rpm of the oscillating motion of said device and/or the length in time of said oscillating motion of said device. In embodiments, the rpm and/or the time of said oscillating motion is selected via a wireless connection using a second device. The wireless connection may use, for example, a Bluetooth standard. In accordance with the device disclosed herein above, the rpm of said oscillating motion may be selected within the range of 1000 to 15000 rpm, preferably 2000 to 12000 rpm, and more preferably 4000 to 8000 rpm.

If the method is applied in a therapeutic method, the method removes hyperkeratotic skin from said subject, which has a skin disease treatable by skin abrasion. For example, said subject may have been diagnosed of having psoriasis, or said subject may suffer from a pathogenic skin callus. In embodiments, the method removes hyperkeratotic skin from said subject, wherein said subject is in need of medical chiropody (medical foot care). In case the device is used in a therapeutic method, the device may be used in steps (b) and/or (d) for percutaneous delivery of an active substance, such as for percutaneous delivery of an active substance in the treatment of actinic keratosis prior to photodynamic therapy (PDT).

Generally, the abrasive product should be attached so that the whole backing pad is covered. As noted herein above, the dimensions of the backing pad and the abrasive product allow to easily spot if the abrasive product is correctly attached. If the abrasive product may not be correctly attached, the device of the present disclosure advantageously allows to remove the pad and re-attach same properly. As noted herein above, this may be achieved using, for example, a hook and loop system for attaching the abrasive product to the backing pad.

In embodiments, it is recommendable to apply user protection equipment, such as gloves, a protective mask, and/or a dust extractor in the operating room (in case the device is not equipped with a dust extractor). Suitable dust extractors are medical vacuum system for removal of the abrasion skin dust.

After use, the powered hand-held device may be cleaned using non-alcoholic agent detergent wipes, and/or the battery is charged.

Finally, the present disclosure also provides a method of producing an abrasive product according to the present disclosure, wherein the method comprises the steps of
(a) laminating a fabric or film layer (A) to an upper side of a foam core layer (B) forming an upper fabric side, and to a lower side of said foam core layer (B) forming a lower fabric side, wherein said fabric layer (A) is each adhered to said fabric core layer (B) by way of an adhesive layer (D), wherein said adhesive layer (D) is a hotmelt glue,
(b) hardening the hotmelt glue by drying;
(c) embedding abrasive particles into a resin, and applying the embedded particles to said upper fabric side to form an abrasive layer (C) comprising abrasive particles;
(d) curing the resin of said abrasive layer C by heating; and
(e) cutting out the abrasive product by punching.

The laminating step (a) may comprise flame lamination or dry lamination with thermoplastic adhesive.

The hardening step (b) may comprise drying the adhesive layer in room temperature for few seconds to 10 minutes, preferably 5-30 seconds.

Embedding abrasive particles in step (c) can be implemented with any known process, such as but not limited to coating. The resin is preferably a solid resin, such as phenol formaldehyde, epoxy, polyimide, alkyd, non-saturated polyester and polyamide resins.

The curing step (d) may comprise heating the resin in 100-200 °C for 12-72 hours, preferably 24 hours. In some embodiments, curing can alternatively be initiated by radiation, electron beams, or chemical additives.

The cutting step (e) may comprise punching the predetermined shape of the abrasive product from a blank by shearing force using a punch and die.

Before the laminating step (a) the layers may be strewed electrostatically to prevent forming wrinkles on the abrasive product.

In embodiments, the fabric forming the lower fabric side comprises one part of a hook and loop system for attaching said pad to a dermabrasive device, in particular wherein the lower fabric side comprises the loop part of said system.

In further embodiments, the punching die used in step (e) has a round shape, in particular a circular shape or an oval shape, preferably a circular shape. The punching die used in step (e) may have an inner diameter in the range of 10 to 100 mm, preferably 20 to 77 mm, more preferably 32 to 40 mm, preferably 32 to 39 mm, more preferably 32 to 38 mm, more preferably 32 to 37 mm, more preferably 32 to 36 mm, more preferably 33 to 35 mm, and most preferably a diameter of about 34 mm at its widest point.

The abrasive product obtained in step (e) may have a thickness of 1 to 10 mm, preferably 4 to 10 mm, preferably a thickness of 5 to 9 mm, more preferably a thickness of 5 to 8 mm, and even more preferably of 6 to 7 mm.

In embodiments, said foam core layer (B) has a thickness of 1 to 10 mm, preferably 3 to 7 mm, preferably a thickness of 4 to 6 mm, and most preferably a thickness of about 5 mm. In embodiments, said foam core layer (B) is essentially made of a polymer foam, preferably a synthetic polymer foam, more preferably a polyurethan foam, such as a polyether polyurethan foam; wherein said foam has a hardness such that it allows adapting the pad to the contour of the skin to be abraded.

The fabric or film layer (A) may each independently have a thickness of less than 1 mm, preferably a thickness selected within the range of 0.25 to 0.9 mm, more preferably a thickness selected within the range of 0.4 to 0.8 mm, such as a thickness selected within the range of 0.5 to 0.7 mm. In embodiments, said fabric layer (A) is a woven or non-woven fabric layer made from one or more synthetic polymer fibers, preferably from polyamide fibers.

The abrasive layer (C) may have a thickness of less than 0.5 mm, in particular a thickness within the range of 0.1 to 0.4 mm, such as a thickness within a range of 0.2 to 0.3 mm. In embodiments, said abrasive particles may have a grit number selected within the range of 80 to 4000 according to FEPA, for example 180 to 1000, in particular 360 to 800; and/or have an average particle size in diameter of 20 to 45 µm. The abrasive particles may be made of any suitable material. In some embodiments, said abrasive particles are made of silicone carbide. The abrasive particles may be fixed to the abrasive layer by way of any suitable means, and/or as described herein above.

The adhesive layer (D) may independently have each a thickness of less than 0.5 mm, in particular a thickness within the range of 0.1 to 0.4 mm, such as a thickness within a range of 0.1 to 0.3 mm. In embodiments, said hotmelt glue of said adhesive layer (D) is a glue made of a mixture of synthetic plastics, preferably a mixture of synthetic plastics based on polyurethane.

## Claims

1. A hand-held, cordless skin abrasion device, comprising
a housing portion (2),
an internal electric drive,
a backing pad (3), and
an attachable abrasive product (4),
and optionally further comprising a dust extractor suitable for the removal of abrasion skin dust,
wherein said backing pad (3) extends from said housing portion (2),
said backing pad (3) is configured to be set in an oscillating and/or random orbital or rotating motion by said electric drive, and
said attachable abrasive product (4) is attached to said backing pad (3) in a mechanical manner, and
said abrasive product (4) has a laminated structure with a foam core layer (B) and a fabric or film layer (A) which is adhered to an upper side of said foam core layer (B) forming an upper fabric or film side of said abrasive product (4), and to a lower side of said foam core layer (B) forming a lower fabric or film side of said abrasive product (4), and
said fabric or film layer (A) is each adhered to said foam core layer (B) by way of an adhesive layer (D), and
only said upper fabric or film side comprises an abrasive layer (C) comprising abrasive particles; and
wherein the abrasive product (4) is attached to the backing pad (3) in an adhesive manner, or wherein the abrasive product (4) is attached to said backing pad (3) using a hook and loop system, wherein the backing pad (3) comprises the hook part of said system, and wherein the abrasive product (4) comprises the loop part of said system positioned at the lower fabric side of said abrasive product (4).

2. The device of claim 1, wherein said device is driven by a battery, in particular wherein the battery is a battery configured to be removed without the requirement for further tools, in particular wherein the battery is a rechargeable battery.

3. The device of claim 1 or 2, wherein a handle portion (1) forms part of the housing portion (2), wherein the handle portion (1) is ergonomically formed, and/or the handle portion (1) and the housing portion (2) are arranged in a gun-like manner.

4. The device of any preceding claim, wherein said backing pad (3) is configured to be set in an oscillating motion of 1000 to 15000 rpm, preferably 2000 to 12000 rpm, and more preferably 4000 to 8000 rpm by said electric drive.

5. The device of any preceding claim, wherein said device comprises means for preselecting the rpm of said oscillating motion and/or the length in time of said oscillating motion, in particular, wherein said means comprise means for wireless communication with a second device and wherein the rpm of said oscillating motion and/or the length in time of said oscillating motion are selected in response to receiving a message defining the rpm of said oscillating motion and/or the length in time of said oscillating motion from said second device via the wireless connection.

6. The device of any preceding claim,wherein the abrasive product (4) is a disposable abrasive product.

7. The device of any preceding claim, wherein the backing pad (3) and the abrasive product (4) each have independently a round shape, in particular a circular shape or an oval shape, preferably a circular shape, most preferably the backing pad (3) and the abrasive product (4) have the same shape;
in particular wherein the backing pad (3) has a diameter in the range of 10 to 100 mm, preferably 20 to 77 mm, more preferably 32 to 40 mm, preferably 32 to 39 mm, more preferably 32 to 38 mm, more preferably 32 to 37 mm, more preferably 32 to 36 mm, more preferably 33 to 35 mm, and most preferably a diameter of about 34 mm at its widest point, and/or wherein the abrasive product (4) has a diameter in the range of 32 to 40 mm, preferably 32 to 39 mm, more preferably 32 to 38 mm, more preferably 32 to 37 mm, more preferably 32 to 36 mm, more preferably 33 to 35 mm, and most preferably a diameter of about 34 mm at its widest point;
in particular wherein the diameter of the backing pad (3) is smaller than the diameter of the abrasive product (4), and wherein the diameter of the backing pad (3) does not differ in more than 4 mm, preferably not more than in 3 mm, more preferably not more than in 2 mm, still more preferably not more than in 1 mm, and most preferably 0,5 mm.

8. The device of any preceding claim, wherein the abrasive product (4) has a thickness of 1 to 10 mm, preferably 4 to 10 mm, preferably a thickness of 5 to 9 mm, more preferably a thickness of 5 to 8 mm, and even more preferably of 6 to 7 mm.

9. The device of any preceding claim, wherein said foam core layer (B) has a thickness of 1 to 10 mm, preferably 3 to 7 mm, preferably a thickness of 4 to 6 mm, and most preferably a thickness of about 5 mm;
and/or wherein said foam core layer (B) is essentially made of a polymer foam, preferably a synthetic polymer foam, more preferably a polyurethan foam, such as a polyether polyurethan foam; wherein said foam has a hardness such that it can adapt to the contour of the skin to be abraded.

10. The device of any preceding claim, wherein said fabric or film layer (A) each independently has a thickness of less than 1 mm, preferably a thickness selected within the range of 0,25 to 0,9 mm, more preferably a thickness selected within the range of 0,4 to 0,8 mm, such as a thickness selected within the range of 0,5 to 0,7 mm;
and/or wherein said fabric layer (A) is a woven or non-woven fabric layer made from one or more synthetic polymer fibers, preferably from polyamide fibers.

11. The device of any preceding claim, wherein said abrasive layer (C) has a thickness of less than 0,5 mm, in particular a thickness within the range of 0,1 to 0,4 mm, such as a thickness within a range of 0,2 to 0,3 mm;
and/or wherein said abrasive layer (C) comprises abrasive particles with a grit number selected within the range of 80 to 4000 according to FEPA, in particular 180 to 1000, in particular 360 to 800, such as 500; and/or abrasive particles with an average particle size in diameter of 3 to 201 µm, in particular 20 to 45 µm;
optionally wherein said abrasive particles in said abrasive layer (C) are made of silicone carbide.

12. The device of any preceding claim, wherein said abrasive particles are embedded in said abrasive layer (C) using a cured resin, in particular thermally cured resin or radiation cured resin.

13. The device of any preceding claim, wherein said adhesive layer (D) has independently each a thickness of less than 0,5 mm, in particular a thickness within the range of 0,1 to 0,4 mm, such as a thickness within a range of 0,1 to 0,3 mm;
and/or wherein said adhesive layer (D) is a cured hotmelt glue, in particular a cured glue made of a mixture of synthetic plastics, preferably a mixture of synthetic plastics based on polyurethane.

14. A non-therapeutic method of abrading skin from at least one subject,
wherein said method comprises the steps of
(a) providing a skin abrasion device as defined in any one of claims 1-13 comprising a first attachable abrasive product with a selected first particle size of the abrasive particles;
(b) conducting a first abrading step, wherein skin is abraded from a subject using the skin abrasion device of step (a);
(c) removing said first attachable abrasive product from said skin abrasion device, and attaching a second attachable abrasive product with a selected second particle size of the abrasive particles; and
(d) conducting a second abrading step, wherein skin is abraded from a subject using the skin abrasion device resulting from step (c);
optionally wherein method steps (c) and (d) are repeated at least one more time.

15. The method of claim 14, wherein the subject in step (b) and step (d) is the same subject;
in particular wherein the second particle size is the same or smaller than the first particle size, preferably wherein the second particle size is smaller than the first particle size.

16. The method of claim 14 or 15, wherein the method further comprises after step (a) and prior to step (b), and/or after step (b) and prior to step (d), preselecting the rpm of the oscillating motion of said device and/or the length in time of said oscillating motion of said device; optionally wherein the rpm and/or the time of said oscillating motion is selected via a wireless connection using a second device, in particular wherein said wireless connection uses a Bluetooth standard;
in particular wherein the rpm of said oscillating motion is elected within the range of 1000 to 15000 rpm, preferably 2000 to 12000 rpm, and more preferably 4000 to 8000 rpm.

## Patentansprüche

1. Handgeführte, kabellose Vorrichtung zum Abreiben von Haut, umfassend
einen Gehäuseabschnitt (2),
einen inneren elektrischen Antrieb,
einen Schleifteller (3), und
ein anbringbares Abriebprodukt (4),
und optional weiter eine Staubabsaugung umfassend, die zum Entfernen von Hautabriebstaub geeignet ist,
wobei
sich der Schleifteller (3) von dem Gehäuseabschnitt (2) erstreckt,
der Schleifteller (3) ausgebildet ist, um durch den elektrischen Antrieb in eine oszillierende und/oder Exzenter- oder Drehbewegung versetzt zu werden, und
das anbringbare Abriebprodukt (4) in mechanischer Weise an dem Schleifteller (3) angebracht ist, und
das Abriebprodukt (4) eine laminierte Struktur mit einer Schaumkernschicht (B) und einer Gewebe- oder Folienschicht (A) aufweist, die an einer Oberseite der Schaumkernschicht (B), wodurch eine obere Gewebe- oder Folienseite des Abriebprodukts (4) gebildet wird, und an einer Unterseite der Schaumkernschicht (B) anhaftet, wodurch eine untere Gewebe- oder Folienseite des Abriebprodukts (4) gebildet wird, und
die Gewebe- oder Folienschicht (A) jeweils mittels einer Klebstoffschicht (D) an der Schaumkernschicht (B) anhaftet, und
nur die obere Gewebe- oder Folienseite eine Abriebschicht (C) umfasst, die Abriebpartikel umfasst; und
wobei das Abriebprodukt (4) in klebender Weise an dem Schleifteller (3) angebracht ist, oder wobei das Abriebprodukt (4) unter Verwendung eines Klettsystems an dem Schleifteller (3) angebracht ist, wobei der Schleifteller (3) den Hakenteil des Systems umfasst und wobei das Abriebprodukt (4) den Schlaufenteil des Systems umfasst, das an der unteren Gewebeseite des Abriebprodukts (4) positioniert ist.

2. Vorrichtung nach Anspruch 1, wobei die Vorrichtung durch eine Batterie angetrieben wird, wobei die Batterie insbesondere eine Batterie ist, die ausgebildet ist, um ohne, dass weitere Werkzeuge erforderlich sind, entfernt zu werden, wobei die Batterie insbesondere eine wiederaufladbare Batterie ist.

3. Vorrichtung nach Anspruch 1 oder 2, wobei ein Griffabschnitt (1) ein Teil des Gehäuseabschnitts (2) bildet, wobei der Griffabschnitt (1) ergonomisch gebildet ist und/oder der Griffabschnitt (1) und der Gehäuseabschnitt (2) in pistolenartiger Weise angeordnet sind.

4. Vorrichtung nach einem vorstehenden Anspruch, wobei der Schleifteller (3) ausgebildet ist, um durch den elektrischen Antrieb in eine oszillierende Bewegung von 1000 bis 15000 U/min, vorzugsweise 2000 bis 12000 U/min und bevorzugter 4000 bis 8000 U/min versetzt zu werden.

5. Vorrichtung nach einem vorstehenden Anspruch, wobei die Vorrichtung Mittel zum Vorwählen der U/min der oszillierenden Bewegung und/oder der zeitlichen Dauer der oszillierenden Bewegung umfasst, wobei die Mittel insbesondere Mittel zur drahtlosen Kommunikation mit einer zweiten Vorrichtung umfassen und wobei die U/min der oszillierenden Bewegung und/oder die zeitliche Dauer der oszillierenden Bewegung als Reaktion auf das Empfangen einer Nachricht, welche die U/min der oszillierenden Bewegung und/oder die zeitliche Dauer der oszillierenden Bewegung definiert, die von der zweiten Vorrichtung über die Funkverbindung empfangen wird, ausgewählt werden.

6. Vorrichtung nach einem vorstehenden Anspruch, wobei das Abriebprodukt (4) ein Einweg-Abriebprodukt ist.

7. Vorrichtung nach einem vorstehenden Anspruch, wobei der Schleifteller (3) und das Abriebprodukt (4) jeweils unabhängig voneinander eine runde Form, insbesondere eine kreisförmige Form oder eine ovale Form, vorzugsweise eine kreisförmige Form aufweisen, der Schleifteller (3) und das Abriebprodukt (4) besonders bevorzugt dieselbe Form aufweisen;
wobei der Schleifteller (3) insbesondere einen Durchmesser im Bereich von 10 bis 100 mm, vorzugsweise 20 bis 77 mm, bevorzugter 32 bis 40 mm, vorzugsweise 32 bis 39 mm, bevorzugter 32 bis 38 mm, bevorzugter 32 bis 37 mm, bevorzugter 32 bis 36 mm, bevorzugter 33 bis 35 mm und besonders bevorzugt einen Durchmesser von etwa 34 mm an seinem breitesten Punkt aufweist, und/oder wobei das Abriebprodukt (4) einen Durchmesser im Bereich von 32 bis 40 mm, vorzugsweise 32 bis 39 mm, bevorzugter 32 bis 38 mm, bevorzugter 32 bis 37 mm, bevorzugter 32 bis 36 mm, bevorzugter 33 bis 35 mm und besonders bevorzugt einen Durchmesser von etwa 34 mm an seinem breitesten Punkt aufweist;
wobei der Durchmesser des Schleiftellers (3) insbesondere kleiner als der Durchmesser des Abriebprodukts (4) ist, und wobei sich der Durchmesser des Schleiftellers (3) um nicht mehr als 4 mm, vorzugsweise um nicht mehr als 3 mm, bevorzugter um nicht mehr als 2 mm, noch bevorzugter um nicht mehr als 1 mm und besonders bevorzugt um 0,5 mm unterscheidet.

8. Vorrichtung nach einem vorstehenden Anspruch, wobei das Abriebprodukt (4) eine Dicke von 1 bis 10 mm, vorzugsweise 4 bis 10 mm, vorzugsweise eine Dicke von 5 bis 9 mm, bevorzugter eine Dicke von 5 bis 8 mm und selbst noch bevorzugter von 6 bis 7 mm aufweist.

9. Vorrichtung nach einem vorstehenden Anspruch, wobei die Schaumkernschicht (B) eine Dicke von 1 bis 10 mm, vorzugsweise 3 bis 7 mm, vorzugsweise eine Dicke von 4 bis 6 mm und besonders bevorzugt eine Dicke von etwa 5 mm aufweist;
und/oder wobei die Schaumkernschicht (B) im Wesentlichen aus einem Polymerschaum, vorzugsweise einem synthetischen Polymerschaum, bevorzugter einem Polyurethanschaum, wie beispielsweise einem Polyether-Polyurethanschaum, hergestellt ist; wobei der Schaum eine Härte aufweist, sodass er sich der Kontur der abzureibenden Haut anpassen kann.

10. Vorrichtung nach einem vorstehenden Anspruch, wobei die Gewebe- oder Folienschicht (A) jeweils unabhängig voneinander eine Dicke von weniger als 1 mm, vorzugsweise eine innerhalb des Bereichs von 0,25 bis 0,9 mm ausgewählte Dicke, bevorzugter eine innerhalb des Bereichs von 0,4 bis 0,8 mm ausgewählte Dicke, wie etwa eine innerhalb des Bereichs von 0,5 bis 0,7 mm ausgewählte Dicke aufweist;
und/oder wobei die Gewebeschicht (A) eine gewebte oder nicht gewebte Gewebeschicht ist, die aus einer oder mehreren synthetischen Polymerfasern, vorzugsweise aus Polyamidfasern, hergestellt ist.

11. Vorrichtung nach einem vorstehenden Anspruch, wobei die Abriebschicht (C) eine Dicke von weniger als 0,5 mm, insbesondere eine Dicke innerhalb des Bereichs von 0,1 bis 0,4 mm, wie beispielsweise eine Dicke innerhalb eines Bereichs von 0,2 bis 0,3 mm aufweist;
und/oder wobei die Abriebschicht (C) Abriebpartikel mit einer nach FEPA innerhalb des Bereichs von 80 bis 4000 ausgewählten Körnungsnummer, insbesondere 180 bis 1000, insbesondere 360 bis 800, wie beispielsweise 500, umfasst; und/oder Abriebpartikel mit einer durchschnittlichen Partikelgröße im Durchmesser von 3 bis 201 µm, insbesondere 20 bis 45 µm;
wobei die Abriebpartikel in der Abriebschicht (C) optional aus Siliziumkarbid hergestellt sind.

12. Vorrichtung nach einem vorstehenden Anspruch, wobei die Abriebpartikel unter Verwendung eines ausgehärteten Harzes, insbesondere thermisch ausgehärteten Harzes oder strahlungsausgehärteten Harzes, in die Abriebschicht (C) eingebettet sind.

13. Vorrichtung nach einem vorstehenden Anspruch, wobei die Klebstoffschicht (D) jeweils unabhängig eine Dicke von weniger als 0,5 mm, insbesondere eine Dicke innerhalb des Bereichs von 0,1 bis 0,4 mm, wie beispielsweise eine Dicke innerhalb eines Bereichs von 0,1 bis 0,3 mm, aufweist;
und/oder wobei die Klebstoffschicht (D) ein ausgehärteter Schmelzklebstoff ist, insbesondere ein ausgehärteter Klebstoff, der aus einem Gemisch synthetischer Kunststoffe, vorzugsweise einem Gemisch aus synthetischen Kunststoffen basierend auf Polyurethan, gebildet ist.

14. Nichttherapeutisches Verfahren zum Abreiben von Haut von mindestens einem Subjekt, wobei das Verfahren die Schritte umfasst zum
(a) Bereitstellen einer Vorrichtung zum Abreiben von Haut nach einem der Ansprüche 1-13, umfassend ein erstes anbringbares Abriebprodukt mit einer ausgewählten ersten Partikelgröße der Abriebpartikel;
(b) Durchführen eines ersten Abriebschritts, wobei Haut unter Verwendung der Vorrichtung zum Abreiben von Haut aus Schritt (a) von einem Subjekt abgerieben wird;
(c) Entfernen des ersten anbringbaren Abriebprodukts von der Vorrichtung zum Abreiben von Haut und Anbringen eines zweiten anbringbaren Abriebprodukts mit einer ausgewählten zweiten Partikelgröße der Abriebpartikel; und
(d) Durchführen eines zweiten Abriebschritts, wobei Haut unter Verwendung der sich aus Schritt (c) ergebenden Vorrichtung zum Abreiben von Haut von einem Subjekt abgerieben wird;
wobei die Verfahrensschritte (c) und (d) optional mindestens ein weiteres Mal wiederholt werden.

15. Verfahren nach Anspruch 14, wobei das Subjekt in Schritt (b) und Schritt (d) dasselbe Subjekt ist;
wobei die zweite Partikelgröße insbesondere dieselbe wie oder kleiner als die erste Partikelgröße ist, wobei die zweite Partikelgröße vorzugsweise kleiner als die erste Partikelgröße ist.

16. Verfahren nach Anspruch 14 oder 15, wobei das Verfahren weiter nach Schritt (a) und vor Schritt (b) und/oder nach Schritt (b) und vor Schritt (d) das Vorwählen der U/min der oszillierenden Bewegung der Vorrichtung und/oder der zeitlichen Dauer der oszillierenden Bewegung der Vorrichtung umfasst; wobei die U/min und/oder die Zeit der oszillierenden Bewegung optional über eine drahtlose Verbindung unter Verwendung einer zweiten Vorrichtung ausgewählt werden, wobei die drahtlose Verbindung insbesondere einen Bluetooth-Standard verwendet;
wobei die U/min der oszillierenden Bewegung insbesondere innerhalb des Bereichs von 1000 bis 15000 U/min, vorzugsweise 2000 bis 12000 U/min und bevorzugter 4000 bis 8000 U/min ausgewählt wird.

## Revendications

1. Dispositif d'abrasion de la peau portatif, sans fil, comprenant
une portion boîtier (2),
un entraînement électrique interne,
un tampon de support (3), et
un produit abrasif (4) pouvant être fixé,
et comprenant facultativement en outre un extracteur de poussières approprié pour l'élimination des poussières cutanées issues de l'abrasion,
dans lequel ledit tampon de support (3) s'étend à partir de ladite portion boîtier (2), ledit tampon de support (3) est conçu pour être mis en mouvement oscillant et/ou orbital aléatoire ou rotatif par ledit entraînement électrique, et
ledit produit abrasif (4) pouvant être fixé est fixé audit tampon de support (3) de manière mécanique, et
ledit produit abrasif (4) présente une structure stratifiée avec une couche centrale en mousse (B) et une couche de tissu ou de film (A) qui est collée à une face supérieure de ladite couche centrale en mousse (B) pour former une face supérieure en tissu ou en film dudit produit abrasif (4), et à une face inférieure de ladite couche centrale en mousse (B) pour former une face inférieure en tissu ou en film dudit produit abrasif (4), et
ladite couche de tissu ou de film (A) est, dans chaque cas, collée à ladite couche centrale en mousse (B) par l'intermédiaire d'une couche adhésive (D), et
seule ladite face supérieure en tissu ou en film comprend une couche abrasive (C) comprenant des particules abrasives ; et
dans lequel le produit abrasif (4) est fixé au tampon de support (3) de manière adhésive, ou dans lequel le produit abrasif (4) est fixé audit tampon de support (3) en utilisant un système à crochets et boucles, dans lequel le tampon de support (3) comprend la partie crochet dudit système, et dans lequel le produit abrasif (4) comprend la partie boucle dudit système positionnée au niveau de la face inférieure en tissu dudit produit abrasif (4).

2. Dispositif selon la revendication 1, dans lequel ledit dispositif est entraîné par une batterie, en particulier dans lequel la batterie est une batterie conçue pour être retirée sans nécessité d'outils supplémentaires, en particulier dans lequel la batterie est une batterie rechargeable.

3. Dispositif selon la revendication 1 ou 2, dans lequel une portion manche (1) forme une partie de la portion boîtier (2), dans lequel la portion manche (1) est formée de manière ergonomique, et/ou la portion manche (1) et la portion boîtier (2) sont agencées en configuration de type pistolet.

4. Dispositif selon une quelconque revendication précédente, dans lequel ledit tampon de support (3) est conçu pour être mis en mouvement oscillant de 1000 à 15 000 tr/min, préférablement de 2000 à 12 000 tr/min, et plus préférentiellement de 4000 à 8000 tr/min par ledit entraînement électrique.

5. Dispositif selon une quelconque revendication précédente, dans lequel ledit dispositif comprend des moyens de présélection des tr/min dudit mouvement oscillant et/ou de la durée dudit mouvement oscillant, en particulier, dans lequel lesdits moyens comprennent des moyens de communication sans fil avec un deuxième dispositif et dans lequel les tr/min dudit mouvement oscillant et/ou la durée dudit mouvement oscillant sont sélectionnés en réponse à la réception d'un message définissant les tr/min dudit mouvement oscillant et/ou la durée dudit mouvement oscillant en provenance dudit deuxième dispositif via la liaison sans fil.

6. Dispositif selon une quelconque revendication précédente, dans lequel le produit abrasif (4) est un produit abrasif jetable.

7. Dispositif selon une quelconque revendication précédente, dans lequel le tampon de support (3) et le produit abrasif (4) présentent chacun indépendamment une forme ronde, en particulier une forme circulaire ou une forme ovale, préférablement une forme circulaire, le plus préférentiellement le tampon de support (3) et le produit abrasif (4) présentent la même forme ;
en particulier dans lequel le tampon de support (3) présente un diamètre dans la plage de 10 à 100 mm, préférablement de 20 à 77 mm, plus préférentiellement de 32 à 40 mm, préférablement de 32 à 39 mm, plus préférentiellement de 32 à 38 mm, plus préférentiellement de 32 à 37 mm, plus préférentiellement de 32 à 36 mm, plus préférentiellement de 33 à 35 mm, et le plus préférentiellement un diamètre d'environ 34 mm en son point le plus large, et/ou dans lequel le produit abrasif (4) présente un diamètre dans la plage de 32 à 40 mm, préférablement de 32 à 39 mm, plus préférentiellement de 32 à 38 mm, plus préférentiellement de 32 à 37 mm, plus préférentiellement de 32 à 36 mm, plus préférentiellement de 33 à 35 mm, et le plus préférentiellement un diamètre d'environ 34 mm en son point le plus large ;
en particulier dans lequel le diamètre du tampon de support (3) est inférieur au diamètre du produit abrasif (4), et dans lequel le diamètre du tampon de support (3) ne diffère pas de plus de 4 mm, préférablement pas de plus de 3 mm, plus préférentiellement pas de plus de 2 mm, plus encore préférentiellement pas de plus de 1 mm, et le plus préférentiellement 0,5 mm.

8. Dispositif selon une quelconque revendication précédente, dans lequel le produit abrasif (4) présente une épaisseur de 1 à 10 mm, préférablement de 4 à 10 mm, préférablement une épaisseur de 5 à 9 mm, plus préférentiellement une épaisseur de 5 à 8 mm, et encore plus préférentiellement de 6 à 7 mm.

9. Dispositif selon une quelconque revendication précédente, dans lequel ladite couche centrale en mousse (B) présente une épaisseur de 1 à 10 mm, préférablement de 3 à 7 mm, préférablement une épaisseur de 4 à 6 mm, et le plus préférentiellement une épaisseur d'environ 5 mm ;
et/ou dans lequel ladite couche centrale en mousse (B) est essentiellement constituée d'une mousse polymère, préférablement d'une mousse polymère synthétique, plus préférentiellement d'une mousse de polyuréthane, telle qu'une mousse de polyuréthane polyéther ; dans lequel ladite mousse présente une dureté telle qu'elle peut s'adapter au contour de la peau à abraser.

10. Dispositif selon une quelconque revendication précédente, dans lequel ladite couche de tissu ou de film (A) présente, dans chaque cas, une épaisseur inférieure à 1 mm, préférablement une épaisseur sélectionnée dans la plage de 0,25 à 0,9 mm, plus préférentiellement une épaisseur sélectionnée dans la plage de 0,4 à 0,8 mm, telle qu'une épaisseur sélectionnée dans la plage de 0,5 à 0,7 mm ;
et/ou dans lequel ladite couche de tissu (A) est une couche de tissu tissé ou non tissé constituée d'une ou de plusieurs fibres de polymère synthétique, préférablement de fibres de polyamide.

11. Dispositif selon une quelconque revendication précédente, dans lequel ladite couche abrasive (C) présente une épaisseur inférieure à 0,5 mm, en particulier une épaisseur dans la plage de 0,1 à 0,4 mm, telle qu'une épaisseur dans une plage de 0,2 à 0,3 mm ;
et/ou dans lequel ladite couche abrasive (C) comprend des particules abrasives avec un numéro de grain sélectionné dans la plage de 80 à 4000 selon la FEPA, en particulier de 180 à 1000, en particulier de 360 à 800, telles que 500 ; et/ou des particules abrasives avec une taille moyenne des particules en diamètre de 3 à 201 µm, en particulier de 20 à 45 µm ;
facultativement dans lequel lesdites particules abrasives dans ladite couche abrasive (C) sont constituées de carbure de silicone.

12. Dispositif selon une quelconque revendication précédente, dans lequel lesdites particules abrasives sont incorporées dans ladite couche abrasive (C) en utilisant une résine durcie, en particulier une résine durcie thermiquement ou une résine durcie par rayonnement.

13. Dispositif selon une quelconque revendication précédente, dans lequel ladite couche adhésive (D) présente indépendamment, dans chaque cas, une épaisseur inférieure à 0,5 mm, en particulier une épaisseur dans la plage de 0,1 à 0,4 mm, telle qu'une épaisseur dans une plage de 0,1 à 0,3 mm ;
et/ou dans lequel ladite couche adhésive (D) est une colle thermofusible durcie, en particulier une colle durcie constituée d'un mélange de matières plastiques synthétiques, préférablement un mélange de matières plastiques synthétiques à base de polyuréthane.

14. Procédé non thérapeutique d'abrasion de la peau d'au moins un sujet, dans lequel ledit procédé comprend les étapes de
(a) fourniture d'un dispositif d'abrasion de la peau tel que défini dans l'une quelconque des revendications 1 à 13 comprenant un premier produit abrasif pouvant être fixé avec une première granulométrie sélectionnée des particules abrasives ;
(b) réalisation d'une première étape d'abrasion, dans lequel de la peau d'un sujet est abrasée en utilisant le dispositif d'abrasion de la peau de l'étape (a) ;
(c) retrait dudit premier produit abrasif pouvant être fixé dudit dispositif d'abrasion de la peau, et fixation d'un deuxième produit abrasif pouvant être fixé avec une deuxième granulométrie sélectionnée des particules abrasives ; et
(d) réalisation d'une deuxième étape d'abrasion, dans lequel de la peau d'un sujet est abrasée en utilisant le dispositif d'abrasion de la peau résultant de l'étape (c) ;
facultativement dans lequel les étapes de procédé (c) et (d) sont répétées au moins une fois supplémentaire.

15. Procédé selon la revendication 14, dans lequel le sujet à l'étape (b) et à l'étape (d) est le même sujet ;
en particulier dans lequel la deuxième granulométrie est identique ou inférieure à la première granulométrie, préférablement dans lequel la deuxième granulométrie est inférieure à la première granulométrie.

16. Procédé selon la revendication 14 ou la revendication 15, dans lequel le procédé comprend en outre, après l'étape (a) et avant l'étape (b), et/ou après l'étape (b) et avant l'étape (d), la présélection des tr/min du mouvement oscillant dudit dispositif et/ou de la durée dudit mouvement oscillant dudit dispositif ; facultativement dans lequel les tr/min et/ou le temps dudit mouvement oscillant sont sélectionnés via une liaison sans fil en utilisant un deuxième dispositif, en particulier dans lequel ladite liaison sans fil utilise une norme Bluetooth ;
en particulier dans lequel les tr/min dudit mouvement oscillant sont sélectionnés dans la plage de 1000 à 15 000 tr/min, préférablement de 2000 à 12 000 tr/min, et plus préférentiellement de 4000 à 8000 tr/min.
